# EUROPEAN PATENT SPECIFICATION

(11) **EP 1 922 315 B1**
(45) Date of publication and mention of the grant of the patent: **27.05.2009**
(21) Application number: 05777481.2
(22) Date of filing: 09.09.2005
(51) Int. Cl.: C07D 405/06

(54) **PREPARATION OF AN ATORVASTATIN INTERMEDIATE**
HERSTELLUNG EINES ATORVASTATIN-ZWISCHENPRODUKTS
PREPARATION D'UN INTERMEDIAIRE DE L' ATORVASTATINE

(43) Date of publication of application: 21.05.2008
(73) Proprietor: Pfizer Science and Technology Ireland Limited, County Dublin (IE)
(72) Inventor: O'SULLIVAN, Susan, Cork (IE); O'NEILL, John, County Cork (IE)
(74) Representative: Rudge, Andrew John
(86) International application number: PCT/IE2005/000094
(87) International publication number: WO 2007/029216

(56) References cited:
- EP-A- 0 247 633
- WO-A-02/43667
- BAUMANN K L ET AL: "THE CONVERGENT SYNTHESIS OF CI-981, AN OPTICALLY ACTIVE, HIGHLY POTENT, TISSUE SELECTIVE INHIBITOR OF HMG-COA REDUCTASE" TETRAHEDRON LETTERS, ELSEVIER, AMSTERDAM, NL, vol. 33, no. 17, 21 April 1992 (1992-04-21), pages 2283-2284, XP000608147 ISSN: 0040-4039 cited in the application
- ROTH B D ET AL: "INHIBITORS OF CHOLESTEROL BIOSYNTHESIS. 3. TETRAHYDRO-4-HYDROXY-6-[2- (1H-PYRROL-1-YL)ETHYL]-2H-PYRAN-2-ONE INHIBITORS OF HMG-COA REDUCTASE. 2. EFFECTS OF INTRODUCING SUBSTITUENTS AT POSITIONS THREE AND FOUR OF THE PYRROLE NUCLEUS" JOURNAL OF MEDICINAL CHEMISTRY, AMERICAN CHEMICAL SOCIETY. WASHINGTON, US, vol. 34, no. 1, 1991, pages 357-366, XP002356812 ISSN: 0022-2623

## Description

### Introduction

The invention relates to a process for preparing atorvastatin lactone. Atorvastatin lactone is a trans-6-[2-(substituted pyrrole-1-yl)alkyl]pyran-2-one which is known by the chemical name (2R-trans)-5-(4-fluorophenyl)-2-(1-methyethyl)-N,4-diphenyl-1-[2-(tetrahydro-4-hydroxy-6-oxo-2H-pyran-2-yl)ethyl]-1H-pyrrole-3-carboxamide.

Atorvastatin lactone is the penultimate intermediate in the preparation of another trans-6-[2-(substituted pyrrole-1-yl)alkyl]pyran-2-one, atorvastatin calcium known by the chemical name [R-R*,R*)]-2-(4-fluorophenyl-(3,8-dihydroxy-5-(1-methylethyl)-3-phenyl-4-[(phenylamino)carbonyl]-1H-pyrrole-1-heptanoic acid hemi calcium salt.

Atorvastatin as well as some of its metabolites is pharmacologically active in humans and is useful as a hypolipidemic and hypocholesterolemic agent. In particular, atorvastatin is useful as a selective and competitive inhibitor of the enzyme 3-hydroxy-3-methylglutaryl-coenzyme A (HMG-CoA) reductase, the rate-limiting enzyme that converts 3-hydroxy-3-methylglutaryl-coenzyme A to mevalonate, a precursor of sterols such as cholesterol. The conversion of HMG-CoA to mevalonate is an early and rate-limiting step in cholesterol biosynthesis.

United States Patent Number 4,681,893 discloses certain trans -6-[2-(3- or 4-carboxamido-substituted-pyrrol -1-yl)alkyl]-4-hydroxy-pyran-2-ones including trans (±)-5-(4-fluorophenyl)-2-(1-methylethyl)-N, 4-diphenyl-1-[(2-tetrahydro-4-hydroxy-6-oxo-2H-pyran-2 - yl)ethyl]-1H-pyrrole-3-carboxamide.

United States Patent Number 5,273,995 discloses the enantiomer having the R form of the ring-opened acid of trans -5-(4-fluorophenyl)-2-(1-methylethyl) N, 4-diphenyl-1-[(2-tetrahydro-4 -hydroxy-6-oxo-2H-pyran-2-yl)ethyl]-1H-pyrrole-3-carboxamide, i.e., [R- (R*,R*)]-2-(4-fluorophenyl)-β, δ-dihydroxy-5-(1-methylethyl)-3-phenyl-4-[(phenylamino) carbonyl]-1H-pyrrole-1-heptanoic acid.

The above described atorvastatin compounds have been prepared by a superior convergent route disclosed in the following United States Patent Numbers 5,003,080; 5,097,045; 5,103,024; 5,124,482 and 5,149,837 and Baumann K.L., Butler D.E., Deering C.F., et al, Tetrahedron Letters 1992;33:2283-2284.

One of the critical intermediates outlined in United States Patent Number 5,097,045 has also been produced using novel chemistry, as described in United States Patent Number 5,155,251 and Brower P.L., Butler D.E., Deering C.F., et al, Tetrahedron Letters 1992;33:2279-2282.

United States Patent Numbers 5,216,174; 5,245,047; 5,248,793; 5,280,126; 5,397,792; 5,342,952; 5,298,627; 5,446,054; 5,470,981; 5,489,690; 5,489,691; 5,5109,488; WO97/03960; WO98/0954 and WO99/32434 disclose various processes and key intermediates for preparing atorvastatin.

The process for preparing atorvastatin lactone is particularly sensitive and vulnerable to the formation of process impurities which may cause product rejection and decreased yields.

The object of the present invention is to provide an improved process for preparing atorvastatin lactone with increased yield and reduced cycle time.

### Statements of Invention

According to the present invention there is provided a process for preparing atorvastatin lactone comprising the steps of:-
hydrogenating tert-butyl isopropylidene nitrile to tert-butyl isopropylidene amine;
condensing tert-butyl isopropylidene amine thus formed with the diketone of atorvastatin to form acetonide ester;
deprotecting the diol protecting acetonide ester to form a diol ester- the acetonide ester being dissolved in methanol and treated with an acid;
saponifying the diol ester to form a sodium salt;
removing methanol from the diol acid sodium salt mixture so that the reaction mixture contains less than 3% w/v of methanol;
reacidifying the sodium salt to the free diol acid; and
forming atorvastatin lactone from the diol acid.

In a preferred embodiment the process includes the step of directly drying the atorvastatin lactone without further purification.

Preferably the methanol is removed by distillation. Vacuum distillation is preferred. However, atmospheric distillation may alternatively be employed.

### Detailed Description

The invention will be more clearly understood from the following description given by way of example only.

The process for preparing atorvastatin lactone, illustrated in scheme 1, comprises the steps of
hydrogenating tert-butyl isopropylidene nitrile to tert-butyl isopropylidene amine using sponge nickel and isopropanol;
acid catalysed Paal-Knorr condensing of tert-butyl isopropylidene amine thus formed with the diketone of atorvastatin to form acetonide ester;
acid-catalysed deprotecting of the diol protecting acetonide ester by dissolving the acetonide ester in methanol and treating with an acid to form a diol ester;
saponifying the diol ester to form a sodium salt;
removing process impurities from the reaction mixture using methyl tert butyl ether;
removing methanol from the reaction mixture by distillation at approximately 70°C under vacuum or approximately 99°C at atmospheric pressure leaving less than 3% w/v of methanol in the reaction mixture;
reacidifying the sodium salt to the free diol acid; and
forming atorvastatin lactone from the diol acid.

The methyl ester of atorvastatin is a major impurity in crude atorvastatin with typical levels at 1 to 1.5%.

We have surprisingly found that by reducing the level of residual methanol present from approximately 10 to 15% to less than 3% w/v, especially levels as high as 2.6%w/v the level of the methyl ester impurity in atorvastatin lactone is reduced to insignificant levels (≤ 0.1%).

In the present invention residual methanol is effectively removed from the reaction mixture using vacuum distillation or distillation at high temperatures before acid is introduced in the reacidification of the sodium salt to the diol acid.

Distillation to remove methanol has been shown to result in a significant decrease in the level of the atorvastatin methyl ester impurity in isolated crude atorvastatin lactone.

Typically, before drying the pure product, recrystallisation of crude atorvastatin lactone from toluene is carried out to remove process impurities such as diol acid, methyl ester and other minor impurities.

However, we have found that the removal of methanol essentially removes the methyl ester impurity from crude lactone and has the potential to increase the product yield in the recrystallisation step as methyl ester impurities are no longer present.

More significantly we have found that removal of the final recrystallisation step is now possible. Eliminating this additional processing step results in an overall increase in yield of approximately 3 to 5% with a significant reduction in the overall atorvastatin lactone preparation time and equipment utilisation.

### Example 1: (Comparative)

50 g tert-butyl isopropylidene (TBIN), prepared as described in Tetrahedron Letters, 1992, 2279, 13.25 g wet sponge nickel catalyst, 28% ammonia solution (137.5 ml) and 375 ml isopropyl alcohol (IPA) are added to a pressure vessel. The mixture is reduced with 50 psi of hydrogen, then filtered and concentrated in vacuo. The resulting oil is dissolved in 250 ml warm toluene, water washed and again concentrated in vacuo to give an amino ester. The amino ester, 85 g 4-fluoro-α-(2-methyl-1-oxopropyl)-γ-oxo-N,β-diphenyl-benzenebutanamide (diketone of atorvastatin), 12.5 g pivalic acid, 137.5 ml tetrahydrofuran (THF) and 137.5 ml hexanes are charged to an argon inerted pressure vessel which is sealed and heated to 75°C for 96 hours. After cooling the solution is diluted with 400 ml methyl tert-butyl ether (MTBE) and washed firstly with dilute aqueous sodium hydroxide followed by dilute aqueous hydrochloric acid. The mixture is then concentrated in vacuo to give an acetonide ester.

The acetonide ester is dissolved in 275 ml warm methanol and aqueous hydrochloric acid (5 g of 37% hydrochloric acid in 75 ml of water) is added. The mixture is stirred at 30°C to produce a diol ester. 100 ml methyl tert-butyl ether and aqueous sodium hydroxide (150 ml of H₂O and 25 g of 50% aqueous sodium hydroxide) are then added and the mixture stirred at 30°C to produce a sodium salt. 600 ml water is added and the mixture washed twice with 437.5 ml methyl tert-butyl ether. Residual methyl tert-butyl ether and some methanol is removed from the aqueous layer by atmospheric distillation to a temperature of 87-90°C. The mixture is stirred at 75-85°C for 18 hours, then cooled, acidified and extracted into 875 ml toluene. The mixture is heated at reflux for 4 hours and water is removed azeotropically. After cooling, the mixture is filtered and washed with toluene. The crude lactone is then recrystallised from toluene and lactone is isolated as an white solid.
Yield: 36 g ; 59.8% from tert-butyl isopropylidene.
Impurity level: crude Methyl ester 1.3 %.
pure Methyl ester 0.6 %.

### Example 2

50 g tert-butyl isopropylidene (TBIN), prepared as described in Tetrahedron Letters, 1992, 2279, 13.25 g wet sponge nickel catalyst, 28% ammonia solution (137.5 ml) and 375 ml isopropyl alcohol (IPA) are added to a pressure vessel. The mixture is reduced with 50 psi of hydrogen, then filtered and concentrated in vacuo. The resulting oil is dissolved in 250 ml warm toluene, water washed and again concentrated in vacuo to give an amino ester. The amino ester, 85 g 4-fluoro-α-(2-methyl-1-oxopropyl)-y-oxo-N,β-diphenyl-benzenebutanamide (diketone of atorvastatin prepared by a method disclosed in United States Patent Number 5,155,251 which is herein incorporated by reference and Bauman K.L, Butler D.E., Deering C.F., et al Tetrahedron Letters 1992;33:2283-2284), 12.5 g pivalic acid, 137.5 ml tetrahydrofuran (THF) and 137.5 ml hexanes are charged to an argon inerted pressure vessel which is sealed and heated to 75°C for 96 hours. After cooling the solution is diluted with 400 ml methyl tert-butyl ether (MTBE) and washed firstly with dilute aqueous sodium hydroxide followed by dilute aqueous hydrochloric acid. The mixture is then concentrated in vacuo to give an acetonide ester.

The acetonide ester is dissolved in 275 ml warm methanol and aqueous hydrochloric acid (5 g of 37% hydrochloric acid in 75 ml of water) is added. The mixture is stirred at 30°C to produce a diol ester. 100 ml methyl tert-butyl ether and aqueous sodium hydroxide (150 ml of H₂O and 25 g of 50% aqueous sodium hydroxide) are then added and the mixture stirred at 30°C to produce a sodium salt. 600 ml water is added and the mixture washed twice with 437.5 ml methyl tert-butyl ether.

In this case, the mixture is distilled under atmospheric pressure to a batch temperature of 70-75°C. A vacuum of approximately -0.25 bar is then applied and distillation is continued until the methanol content of the mixture is reduced to less than 2.6%w/v. The batch is stirred at 75-85°C for 18 hours, then cooled, acidified and extracted into 875 ml toluene. The mixture is heated at reflux for 4 hours and water removed azeotropically. After cooling the mixture is filtered, washed with toluene and dried directly. Lactone is isolated as awhite solid.
Yield: 37.9 g ; 63% from tert-butyl isopropylidene.
Impurity level: Methyl ester 0.16%.

### Example 3

50 g tert-butyl isopropylidene (TBIN), prepared as described in Tetrahedron Letters, 1992, 2279, 13.25 g wet sponge nickel catalyst, 28% ammonia solution (137.5 ml) and 375 ml isopropyl alcohol (IPA) are added to a pressure vessel. The mixture is reduced with 50 psi of hydrogen, then filtered and concentrated in vacuo. The resulting oil is dissolved in 250 ml warm toluene, water washed and again concentrated in vacuo to give an amino ester. The amino ester, 85 g 4-fluoro-α-(2-methyl-1-oxopropyl)-γ-oxo-N,β-diphenyl-benzenebutanamide (diketone of atorvastatin prepared by a method disclosed in United States Patent Number 5,155,251 which is herein incorporated by reference and Bauman K.L, Butler D.E., Deering C.F., et al Tetrahedron Letters 1992;33:2283-2284), 12.5 g pivalic acid, 137.5 ml tetrahydrofuran (THF) and 137.5 ml hexanes are charged to an argon inerted pressure vessel which is sealed and heated to 75°C for 96 hours. After cooling the solution is diluted with 400 ml methyl tert-butyl ether (MTBE) and washed firstly with dilute aqueous sodium hydroxide followed by dilute aqueous hydrochloric acid. The mixture is then concentrated in vacuo to give an acetonide ester.

The acetonide ester is dissolved in 275 ml warm methanol and aqueous hydrochloric acid (5 g of 37% hydrochloric acid in 75 ml of water) is added. The mixture is stirred at 30°C to produce a diol ester. 100 ml methyl tert-butyl ether and aqueous sodium hydroxide (150 ml of H₂O and 25 g of 50% aqueous sodium hydroxide) are then added and the mixture stirred at 30°C to produce a sodium salt. 600 ml water is added and the mixture washed twice with 437.5 ml methyl tert-butyl ether.

In this case, the mixture is distilled under atmospheric pressure to a batch temperature of 99°C. Distillation is continued until the methanol content of the mixture is reduced to 0.4w/v. The batch is stirred at 75-85°C for 18 hours, then cooled, acidified and extracted into 875 ml toluene. The mixture is heated at reflux for 4 hours and water is remove azeotropically. After cooling, the mixture is filtered, washed with toluene and dried directly. Lactone is isolated as awhite solid.
Yield: 37.9 g ;63% from tert-butyl isopropylidene.
Impurity level: Methyl ester 0.1%.

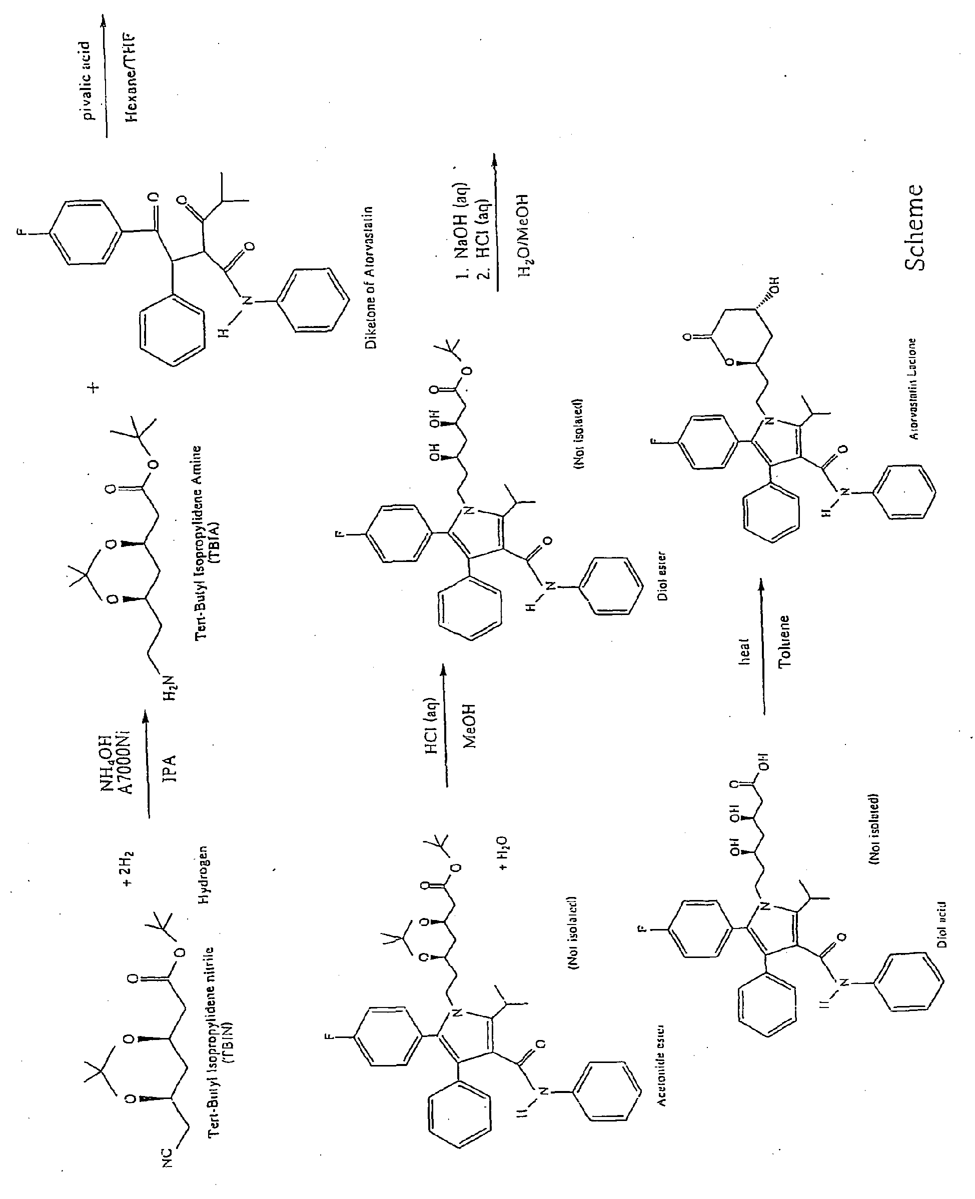

## Claims

1. A process for preparing atorvastatin lactone comprising the steps of:-
hydrogenating tert-butyl isopropylidene nitrile to tert-butyl isopropylidene amine;
condensing tert-butyl isopropylidene amine thus formed with the diketone of atorvastatin to form acetonide ester;
deprotecting the diol protecting acetonide ester to form a diol ester by dissolving the acetonide ester in methanol and treating with an acid;
saponifying the diol ester to form a sodium salt;
removing methanol from the diol acid sodium salt reaction mixture so that the reaction mixture includes less than 3% w/v of methanol;
reacidifying the sodium salt to the free diol acid; and
forming atorvastatin lactone from the diol acid.

2. A process as claimed in claim 1 including the step of directly drying the atorvastatin lactone without further purification.

3. A process as claimed in claim 1 or 2 wherein methanol is removed from the diol acid sodium salt reaction mixture by distillation.

4. A process as claimed in claim 3 wherein methanol is removed from the diol acid sodium salt reaction mixture by vacuum distillation.

5. A process as claimed in claim 3 wherein the methanol is removed from the diol acid sodium salt reaction mixture by atmospheric distillation.

## Patentansprüche

1. Verfahren zur Herstellung von Atorvastatin-Lacton, umfassend die Schritte:
Hydrieren von tert-Butylisopropylidennitril zu tert-Butylisopropylidenamin;
Kondensieren von tert-Butylisopropylidenamin, das so gebildet wurde, mit dem Diketon von Atorvastatin unter Bildung von Acetonidester;
Entschützen des diolschützenden Acetonidesters unter Bildung eines Diolesters durch Lösen des Acetonidesters in Methanol und Behandeln mit einer Säure;
Verseifen des Diolesters unter Bildung eines Natriumsalzes;
Entfernen von Methanol aus dem Diol-Säure-Natriumsalz-Reaktionsgemisch, so dass das Reaktionsgemisch weniger als 3% G/V Methanol umfasst;
erneutes Azidifizieren des Natriumsalzes zu der freien Diolsäure und
Bilden von Atorvastatin-Lacton aus der Diolsäure.

2. Verfahren, wie es in Anspruch 1 beansprucht ist, das den Schritt des direkten Trocknens des Atorvastatin-Lactons ohne weitere Reinigung umfasst.

3. Verfahren, wie es in Anspruch 1 oder 2 beansprucht ist, wobei Methanol aus dem Diolsäure-Natriumsalz-Reaktionsgemisch durch Destillation entfernt wird.

4. Verfahren, wie es in Anspruch 3 beansprucht ist, wobei Methanol aus dem Diolsäure-Natriumsalz-Reaktionsgemisch durch Vakuumdestillation entfernt wird.

5. Verfahren, wie es in Anspruch 3 beansprucht ist, wobei das Methanol aus dem Diolsäure-Natriumsalz-Reaktionsgemisch durch atmosphärische Destillation entfernt wird.

## Revendications

1. Procédé pour préparer la lactone de l'atorvastatine, comprenant les étapes suivantes :
- hydrogénation du nitrile de tert-butyl-isopropylidène en amine de tert-butyl-isopropylidène ;
- condensation de l'amine de tert-butyl-isopropylidène ainsi formée avec la dicétone d'atorvastatine pour former un ester d'acétonide ;
- élimination de l'ester d'acétonide protégeant le diol, par dissolution de l'ester d'acétonide dans du méthanol et traitement avec un acide, pour former un diol-ester ;
- saponification du diol-ester pour former un sel de sodium ;
- élimination du méthanol depuis le mélange réactionnel contenant le sel sodique du diol-acide de telle sorte que le mélange réactionnel inclut moins de 3 % (poids/volume) de méthanol ;
- réacidification du sel sodique pour obtenir le diol-acide libre ; et
- formation de la lactone d'atorvastatine à partir du diol-acide.

2. Procédé tel que revendiqué dans la revendication 1, incluant l'étape de séchage direct de la lactone d'atorvastatine sans autre purification.

3. Procédé tel que revendiqué dans la revendication 1 ou 2, dans lequel le méthanol est éliminé, par distillation, du mélange réactionnel contenant le sel sodique du diol-acide.

4. Procédé tel que revendiqué dans la revendication 3, dans lequel le méthanol est éliminé, par distillation sous vide, du mélange réactionnel contenant le sel sodique du diol-acide.

5. Procédé tel que revendiqué dans la revendication 3, dans lequel le méthanol est éliminé, par distillation atmosphérique, du mélange réactionnel contenant le sel sodique du diol-acide.
